# EUROPÄISCHE PATENTSCHRIFT

(11) **EP 1 678 127 B1**
(45) Veröffentlichungstag und Bekanntmachung des Hinweises auf die Patenterteilung: **25.07.2007**
(21) Anmeldenummer: 04790384.4
(22) Anmeldetag: 14.10.2004
(51) Int. Cl.: C07C 319/14, C07C 323/22

(54) **VERFAHREN ZUR HERSTELLUNG VON ALPHA-(3-ARYLTHIO)-ACETOPHENONEN**
METHOD FOR THE PRODUCTION OF ALPHA-(3-ARYLTHIO)-ACETOPHENONES
PROCEDE POUR REALISER DES ALPHA-(3-ARYLTHIO)-ACETOPHENONES

(30) Priorität: 20.10.2003 DE 10349249
(43) Veröffentlichungstag der Anmeldung: 12.07.2006
(73) Patentinhaber: BASF Aktiengesellschaft, 67056 Ludwigshafen (DE)
(72) Erfinder: ALTMAYER, Marco, 68549 Ilvesheim (DE); SIEGEL, Wolfgang, 67117 Limburgerhof (DE)
(86) Internationale Anmeldenummer: PCT/EP2004/011521
(87) Internationale Veröffentlichungsnummer: WO 2005/042477

(56) Entgegenhaltungen:
- US-A- 4 133 814
- N.E. MACKENZIE, ET AL.: "New dyes based on 3-aryl-benzo- and -naphtho-1,4-thiazines" JOURNAL OF THE CHEMICAL SOCIETY, PERKIN TRANSACTIONS 1, Nr. 12, Dezember 1980 (1980-12), Seiten 2923-2932, XP002319221 ROYAL SOCIETY OF CHEMISTRY, LETCHWORTH, GB

## Beschreibung

Die vorliegende Erfindung betrifft ein verbessertes Verfahren zur Herstellung von α-(3-Arylthio)-acetophenonen der allgemeinen Formel 1

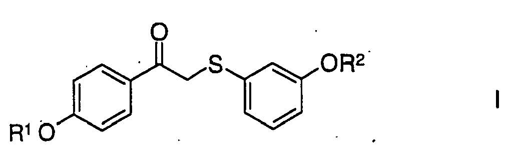

in der die Substituenten R¹ und R² unabhängig voneinander für C₁-C₆-Alkyl, SiR³₃, wobei der Substituent R³ für einen C₁-C₆-Alkylrest steht, oder einen gegebenenfalls substituierten Phenyl- oder Benzylrest stehen.

Die Verbindungen der Formel I sind wertvolle Zwischenverbindungen bei der Synthese von pharmazeutisch aktiven Substanzen; 1-(4-Methoxyphenyl)- 2-[3-methoxyphenyl)-thio]ethanon (R¹, R² = Methyl) ist ein Baustein zur Herstellung des Anti-Osteoporose-Wirkstoffs Raloxifen.

Verschiedene Methoden zur Herstellung von Acetophenonen der allgemeinen Formel I sind bekannt. Ausgangsverbindungen sind in den meisten Fällen die Acetophenone der allgemeinen Formel II

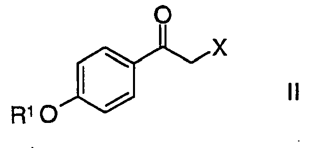

in der der Substituent X für Chlor oder Brom steht und der Substiuent R¹ die oben angegebene Bedeutung hat. Diese Verbindungen werden mit einem Thiol umgesetzt
- in einem Zweiphasensystem aus Essigsäureethylester und Kalilauge (WO 02/42261)
- in einem Ethanol/Wasser/Essigsäureethylester-Gemisch mit Kaliumhydroxid (Tetrahedron Letters 40 (1999) 2909)
- in Ethanol mit Kalilauge (US 4,133,814)
- in einem Ethanol/Wasser-Gemisch mit Kalilauge (US 4,418,068).

Die Ausbeute für das besonders gesuchte 1-(4-Methoxyphenyl)- 2-[3-methoxyphenyl)-thio]ethanon beträgt nach diesen Methoden maximal 86 %. Es bestand daher ein Bedürfnis ein Verfahren bereit zu stellen, das eine höhere Ausbeute an Wertprodukt ermöglicht.

Diese Aufgabe wurde dadurch gelöst, indem man Acetophenone der allgemeinen Formel II 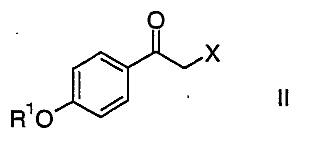 in der der Substituent X für Cl oder Br steht und der Substituent R¹ für C₁-C₆-Alkyl, SiR³₃, wobei der Substituent R³ für einen C₁-C₆-Alkylrest steht, oder einen gegebenenfalls substituierten Phenyl- oder Benzylrest steht, mit einem Thiolat der allgemeinen Formel III 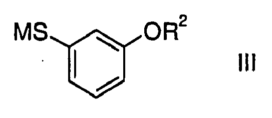 in der M für ein Alkalimetall steht, in Methanol umsetzt.

Das erfindungsgemäße Verfahren dient zur Herstellung von Verbindungen der allgemeinen Formel I, bevorzugt von 1-(4-Methoxyphenyl)- 2-[3-methoxyphenyl)thio]-ethanon.

Als eine Ausgangsverbindung dienen Chlor- oder Bromacetophenone der allgemeinen Formel II, in der der Substituent R¹ für C₁-C₆-Alkyl wie Methyl, Ethyl, iso-Propyl, n-Butyl oder iso-Butyl, Phenyl oder Benzyl steht, wobei die Phenyl- und Benzylreste unter den Reaktionsbedingungen inerte Substituenten wie zum Beispiel Halogen oder Oxyalkyl tragen können, oder R¹ für Tri(C₁-C₆)alkylsilylgruppen wie bevorzugt Trimethylsilyl steht. Bevorzugt werden für R¹ kurzkettige Alkylreste, insbesondere Methyl. Diese Verbindungen sind in an sich bekannter Weise zugänglich, beispielsweise durch Umsetzung der Acetophenone mit Sulfurylchlorid (US 5,710,341) oder mit Brom (Chem. Ber. 1953, 86, 1556).

Die Acetophenone der allgemeinen Formel II werden mit einem Thiolat der allgemeinen Formel III umgesetzt, in der der Substituent R² für C₁-C₆-Alkyl wie Methyl, Ethyl, iso-Propyl, n-Butyl oder iso-Butyl, Phenyl oder Benzyl steht, wobei die Phenyl- und Benzylreste unter den Reaktionsbedingungen inerte Substituenten wie zum Beispiel Halogen oder Oxyalkyl tragen können, oder der Substituent R² für Tri(C₁-C₆)alkyl-silylgruppen wie bevorzugt Trimethylsilyl steht. Bevorzugt werden für R² kurzkettige Alkylreste, insbesondere Methyl.

Das Thiolatkation M steht für Alkalimetalle wie Lithium, Natrium und Kalium.
Die Thiolate können durch Deprotonierung der entsprechenden Thiole hergestellt werden. Dazu werden die Thiole mit einer Base, deren Basenstärke zur Deprotonierung des Thiols ausreichend ist, umgesetzt. Dies kann in einer gesonderten Reaktion unter Isolierung des Thiolats geschehen, bevorzugt ist jedoch eine in situ-Herstellung des Thiolats und anschließende Umsetzung zu Acetophenonen der allgemeinen Formel 1. Bevorzugt für die in situ Herstellung der Thiolate sind als Basen Alkalimetallhydroxide wie Kaliumhydroxid und Natriumhydroxid, Hydride wie Lithiumhydrid und Natriumhydrid, Amide wie Lithium-, Natrium- und Kaliumamid und Alkoholate wie Natrium- und Kaliummethylat. Natriummethylat ist besonders bevorzugt.

Die Umsetzung der Chlor- oder Bromacetophenone der allgemeinen Formel II mit einem Thiolat der allgemeinen Formel III läuft in Methanol ab. Das Methanol kann auch geringe Mengen weiterer polarer Lösungsmittel wie Wasser, jedoch bevorzugt nicht mehr als 5 Gew.% davon enthalten.

Die molaren Verhältnisse der Ausgangsverbindungen betragen im allgemeinen 0,8 bis 2,0 mol Thiolat der allgemeinen Formel III pro Mol der Chlor- oder Bromacetophenon der allgemeinen Formel II, bevorzugt 0,90 bis 1,05 mol pro Mol.

Die Umsetzung kann beispielsweise in einem Rührkessel vorgenommen werden. Bevorzugt wird das Chlor- oder Bromacetophenon der allgemeinen Formel II in Methanol vorgelegt.

Die Menge an Methanol beträgt im Allgemeinen 100- 1000 g, bezogen auf 100 g des eingesetzten Acetophenons der allgemeinen Formel II, bevorzugt 150 - 200 g. Bevorzugt dosiert man dazu das Thiolat der allgemeinen Formel III in Methanol, wobei auf 100 g eingesetztes Thiophenol 100 - 1000 g Methanol, bevorzugt 150 - 200 g eingesetzt werden.

Die Reaktion kann bei Normaldruck und einer Temperatur von bevorzugt 0 bis 50°C durchgeführt werden. Das Ende der Reaktion kann beispielsweise gaschromatographisch festgestellt werden.

Die gesuchten Wertprodukte der allgemeinen Formel I sind in Methanol nur schlecht löslich und fallen daher bei der Umsetzung als Feststoff an. Sie können in einfacher Weise durch Filtration isoliert werden. Das in der Umsetzung gebildete und ausgefallene Alkalimetallchlorid bzw. -bromid kann leicht durch Waschen mit Wasser entfernt werden.

Das erfindungsgemäße Verfahren erlaubt die Herstellung von Verbindungen der allgemeinen Formel in hoher Ausbeute und ist darüber hinaus in verfahrenstechnisch einfacher Weise durchzuführen.

### Beispiel 1

### Herstellung von 1-(4-Methoxyphenyl)- 2-[3-methoxyphenyl)thio]ethanon

216 g (1,54 mol) 3-Methoxythiophenol wurden in einer 2l-Rührapparatur in 253 g (320 ml) Methanol vorgelegt. Bei einer Temperatur von maximal 35°C wurden 275 g (1,51 mol) einer 30%igen methanolischen Natriummethylatlösung zugetropft. Im Anschluss wurden weitere 127 g (1-60 ml) Methanol zum Ansatz gegeben.
Zu 285 g (1,54 mol) Chlormethoxyacetophenon in 494 g (624 ml) Methanol in einem 5l-Rührkolben wurde die oben beschriebene 3-Methoxythiophenolat-Lösung bei maximal 35°C getropft. Der Ansatz wurde 10 Minuten bei Eigentemperatur und danach 1 h bei 0°C gerührt. Das Kristallisat wurde abgesaugt, mit 1,5 l Wasser salzfrei gewaschen und mit 928 ml Methanol nachgewaschen. Das farblose Produkt wurde im Vakuum bei 30°C getrocknet.
Ausbeute: 424 g (1,47 mol): 97,4 % mit einer Reinheit von 99,3 % (GC-Flächen-%)

## Patentansprüche

1. Verfahren zur Herstellung von α-(3-Arylthio)-acetophenonen der allgemeinen Formel I 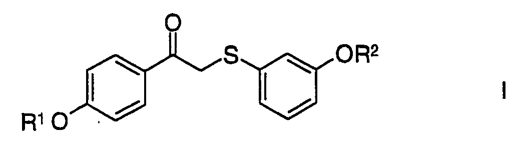 in der die Substituenten R¹ und R² unabhängig voneinander für C₁-C₆-Alkyl, SiR³₃, wobei der Substituent R³ für einen C₁-C₆-Alkylrest steht, oder einen gegebenenfalls substituierten Phenyl- oder Benzylrest stehen, **dadurch gekennzeichnet, dass** man Acetophenone der allgemeinen Formel II 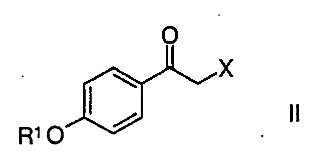 in der der Substituent X für Cl oder Br steht, mit einem Thiolat der allgemeinen Formel III 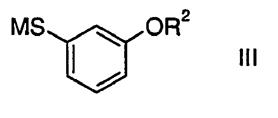 in der M für ein Alkalimetall steht, in Methanol umsetzt.

2. Verfahren nach Anspruch 1, **dadurch gekennzeichnet, dass** man 1-(4-Methoxyphenyl)- 2-[3-methoxyphenyl)thio]ethanon herstellt.

3. Verfahren nach Anspruch 1 oder 2, **dadurch gekennzeichnet, dass** M für Natrium steht.

4. Verfahren nach einem der Ansprüche 1 bis 3, **dadurch gekennzeichnet, dass** man ein Thiolat der allgemeinen Formel III durch Umsetzung des entsprechenden Thiols mit Natriummethylat herstellt.

## Claims

1. A process for preparing α-(3-arylthio)acetophenones of the general formula I 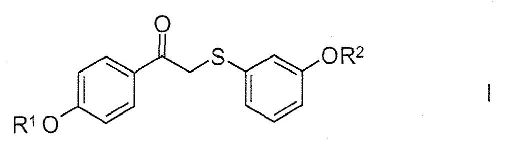 in which the substituents R¹ and R² are each independently C₁-C₆-alkyl, SiR³₃ where the substituent R³ is a C₁-C₆-alkyl radical, or an optionally substituted phenyl or benzyl radical, which comprises reacting, in methanol acetophenones of the general formula II 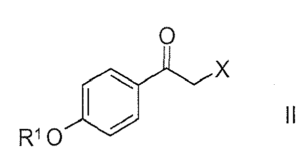 in which the substituent X is Cl or Br with a thiolate of the general formula III 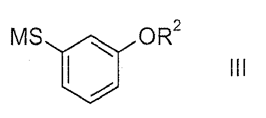 in which M is an alkali metal.

2. The process according to claim 1, wherein 1-(4-methoxyphenyl)-2-[(3-methoxyphenyl)thio]ethanone is prepared.

3. The process according to claim 1 or 2, wherein M is sodium.

4. The process according to any of claims 1 to 3, wherein a thiolate of the general formula III is prepared by reacting the appropriate thiol with sodium methoxide.

## Revendications

1. Procédé de préparation d'α-(3-arylthio)-acétophénones de formule générale I : 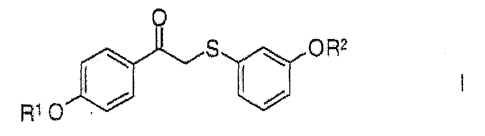 dans laquelle les substituants R¹ et R² représentent, indépendamment l'un de l'autre, un groupe alkyle en C₁-C₆, SiR³₃, le substituant R³ représentant un radical alkyle en C₁-C₆, ou un radical benzyle ou phényle éventuellement substitué, **caractérisé en ce qu'**on fait réagir dans du méthanol des acétophénones de formule générale II : 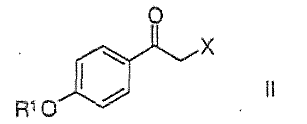 dans laquelle le substituant X représente Cl ou Br, avec un thiolate de formule générale III : 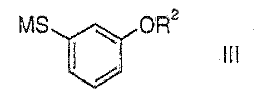 dans laquelle M représente un métal alcalin.

2. Procédé suivant la revendication 1, **caractérisé en ce qu'**on prépare de la 1-(4-méthoxyphényl)-2-[(3-méthoxyphényl)thio]-éthanone.

3. Procédé suivant la revendication 1 ou 2, **caractérisé en ce que** M représente du sodium.

4. Procédé suivant l'une des revendications 1 à 3, **caractérisé en ce qu'**on prépare un thiolate de formule générale III par réaction du thiol correspondant avec un méthylate de sodium.
